(19) Europäisches Patentamt
European Patent Office
Office européen des brevets

(11) EP 2 987 816 A1

(12) # EUROPEAN PATENT APPLICATION
published in accordance with Art. 153(4) EPC

(43) Date of publication:
**24.02.2016 Bulletin 2016/08**

(21) Application number: **14785688.4**

(22) Date of filing: **17.04.2014**

(51) Int Cl.:
***C08G 64/14*** *(2006.01)* ***C07C 39/06*** *(2006.01)*
***F21S 2/00*** *(2016.01)* ***G02F 1/1333*** *(2006.01)*

(86) International application number:
**PCT/JP2014/060926**

(87) International publication number:
**WO 2014/171509 (23.10.2014 Gazette 2014/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.04.2013 JP 2013088408**
**19.04.2013 JP 2013088426**

(71) Applicant: **Idemitsu Kosan Co., Ltd**
**Tokyo 100-8321 (JP)**

(72) Inventors:
- **MARUYAMA, Junpei**
**Sodegaura-shi**
**Chiba 299-0205 (JP)**
- **TASHIRO, Hironori**
**Sodegaura-shi**
**Chiba 299-0205 (JP)**
- **MIYAGAWA, Toshifumi**
**Sodegaura-shi**
**Chiba 299-0205 (JP)**
- **SHIBATA, Masayuki**
**Sodegaura-shi**
**Chiba 299-0205 (JP)**
- **YAMADA, Aki**
**Ichihara-shi**
**Chiba 299-0107 (JP)**
- **SUGA, Koichi**
**Ichihara-shi**
**Chiba 299-0107 (JP)**
- **NAKAYAMA, Yumi**
**Ichihara-shi**
**Chiba 299-0107 (JP)**

(74) Representative: **Hoffmann Eitle**
**Patent- und Rechtsanwälte PartmbB**
**Arabellastraße 30**
**81925 München (DE)**

(54) **POLYCARBONATE RESIN FOR LIQUID CRYSTAL MEMBERS, POLYCARBONATE RESIN COMPOSITION FOR LIQUID CRYSTAL MEMBERS WHICH CONTAINS SAME, AND LIQUID CRYSTAL MEMBER**

(57) Provided is a polycarbonate resin for a liquid crystal member, which is produced by using an end terminator containing 3-pentadecylphenol obtained from a natural product, and which has a YI value of 1.1 or less or a light transmittance at a wavelength of 400 nm of 85% or more.

EP 2 987 816 A1

## Description

### TECHNICAL FIELD

The present invention relates to a polycarbonate resin for a liquid crystal member, a polycarbonate resin composition for a liquid crystal member containing the resin, and a liquid crystal member obtained by molding the resin or the composition, and more specifically, to a polycarbonate resin for a liquid crystal member, which is produced by using a specific phenol compound obtained from a natural product, and which is excellent in flowability and color tone, a polycarbonate resin composition for a liquid crystal member containing the resin, and a liquid crystal member obtained by molding the resin or the composition.

### BACKGROUND ART

A polycarbonate resin has excellent features such as transparency, heat resistance, and a mechanical characteristic, and hence has been used in a wide variety of applications such as housings for OA equipment and home electrical appliances, members in electrical and electronic fields, and optical materials such as a lens. Meanwhile, in recent years, to satisfy demands for the thinning and upsizing of a molded article, and an improvement in molding cycle, it has become necessary to further improve the flowability of the polycarbonate resin. A method involving using a plasticizer or using a resin excellent in flowability such as a styrene-based resin, e.g., ABS, HIPS, or AS has been employed as a method of improving the flowability of the polycarbonate resin. Such method can improve the flowability of the polycarbonate resin, but has involved a problem in that the method reduces excellent impact resistance intrinsic to the polycarbonate resin.

In addition, it has been known that to avoid the problem, the flowability is improved by changing the structure of the polycarbonate resin itself. The following method has been known as one example of such method. A monohydric phenol having a long-chain alkyl group is used as an end terminator, and the flowability is improved by introducing the long-chain alkyl end group into an end of the polycarbonate resin. For example, in Patent Document 1, there is a disclosure that an alkylphenol, carboxylic acid, or acid halide having an alkyl group having 8 to 20 carbon atoms is used as an end terminator. However, only the case of using an acid chloride having an alkyl group having 9 to 17 carbon atoms is disclosed in Examples of Patent Document 1. In addition, in Patent Document 2, there is a disclosure that a polycarbonate having a m-pentadecylphenoxy end group is used as an optical recording medium, but there is no disclosure that the polycarbonate can be used in a liquid crystal member, in particular, a liquid crystal member to be used in thinned liquid crystal equipment.

### CITATION LIST

### PATENT DOCUMENT

Patent Document 1: JP 52-50078 B2

Patent Document 2: JP 2003-041011 A

### SUMMARY OF INVENTION

### TECHNICAL PROBLEM

An obj ect of the present invention is to provide a polycarbonate for a liquid crystal member, which is produced by using an end terminator containing 3-pentadecylphenol obtained from a natural product, in particular, a polycarbonate for a liquid crystal member that can be suitably used in a thin-wall portion.

### SOLUTION TO PROBLEM

<1> A polycarbonate resin for a liquid crystal member, which is produced by using an end terminator comprising 3-pentadecylphenol obtained from a natural product, the polycarbonate resin having a YI value in the following measurement method of 1.1 or less: <Method of measuring YI Value>

500 ppm by mass of bis(2,6-di-t-butyl-4-methylphenyl)pentaerythritol phosphite is added to the polycarbonate resin, the mixture is melted, kneaded, and extruded with a vented single screw extruder of 40 mmφ at a resin temperature of 280°C and a screw rotation speed of 100 rpm to provide a pellet, the resultant pellet is molded into a molded article having a thickness of 3 mm, and the molded article is subjected to measurement with a spectrophotometer by a transmission method at a measurement area of 30 φ under a C2 light source.
<2> A polycarbonate resin for a liquid crystal member, which is produced by using an end terminator comprising 3-pentadecylphenol obtained from a natural product, the polycarbonate resin having a light transmittance at a wavelength of 400 nm in the following measurement method of 85% or more:

<Method of measuring Light Transmittance at Wavelength of 400 nm>

500 ppm by mass of bis(2,6-di-t-butyl-4-methylphenyl)pentaerythritol phosphite is added to the polycarbonate resin, the mixture is melted, kneaded, and extruded with a vented single screw extruder of 40 mmφ at a resin temperature of 280°C and a screw rotation speed of 100 rpm to provide a pellet, the resultant pellet is molded into a moldedarticle having a thickness of 3 mm, and the molded article is subjected to measurement of a total light transmittance with a spectral photometer.
<3> The polycarbonate resin for a liquid crystal member according to the item <1> or <2>, wherein a composition amount of an end group derived from OH with respect to all end groups in the polycarbonate resin is 5.0 mol% or less.
<4> The polycarbonate resin for a liquid crystal member according to any one of the items <1> to <3>, wherein the 3-pentadecylphenol has a purity of 97.5 mass% or more.
<5> The polycarbonate resin for a liquid crystal member according to any one of the items <1> to <3> wherein the 3-pentadecylphenol has a purity of 97.75 mass% or more.
<6> The polycarbonate resin for a liquid crystal member according to any one of the items <1> to <3>, wherein the 3-pentadecylphenol has a purity of 99.33 mass% or more.
<7> The polycarbonate resin for a liquid crystal member according to any one of the items <1> to <6>, wherein the polycarbonate resin is produced by using an end terminator containing 3 -pentadecylphenol obtained by performing distillation and crystallization.
<8> The polycarbonate resin for a liquid crystal member according to the item <7>, wherein the crystallization is performed after the distillation.
<9> The polycarbonate resin for a liquid crystal member according to the item <7> or <8>, wherein the crystallization comprises using a hydrocarbon-based solvent.
<10> The polycarbonate resin for a liquid crystal member according to the item <9>, wherein the hydrocarbon-based solvent to be used in the crystallization comprises one or more of hexane and heptane.
<11> The polycarbonate resin for a liquid crystal member according to any one of the items <7> to <10>, wherein the crystallization comprises using a solvent in an amount of 2 parts by mass or more and 20 parts by mass or less with respect to 1 part by mass of the 3-pentadecylphenol.
<12> The polycarbonate resin for a liquid crystal member according to any one of the items <7> to <10>, wherein the crystallization comprises using a solvent in an amount of 4 parts by mass or more and 10 parts by mass or less with respect to 1 part by mass of the 3-pentadecylphenol.
<13> The polycarbonate resin for a liquid crystal member according to any one of the items <1> to <12>, wherein the end terminator further comprises p-t-butylphenol or p-cumylphenol.
<14> A polycarbonate resin composition for a liquid crystal member, comprising: the polycarbonate resin for a liquid crystal member of any one of the items <1> to <13>; and an aromatic polycarbonate resin except the polycarbonate resin.
<15> A liquid crystal member, which is produced by molding the polycarbonate resin for a liquid crystal member of any one of the items <1> to <13>, or the polycarbonate resin composition for a liquid crystal member of the item <14>.
<16> A polycarbonate resin for a light-guiding plate, wherein the polycarbonate resin for a liquid crystal member of any one of the items <1> to <13> is used for a light-guiding plate.
<17> A polycarbonate resin composition for a light-guiding plate, comprising: the polycarbonate resin for a light-guiding plate of the item <16>; and an aromatic polycarbonate resin except the polycarbonate resin.
<18> A light-guiding plate, which is produced by molding the polycarbonate resin for a light-guiding plate of the item <16> or the polycarbonate resin composition for a light-guiding plate of the item <17>.
<19> A method of producing a polycarbonate resin for a liquid crystal member, comprising using 3-pentadecylphenol obtained from a natural product and having a purity of 97.5 mass% or more as an end terminator.
<20> The method of producing a polycarbonate resin for a liquid crystal member according to the item <19>, wherein the 3-pentadecylphenol has a purity of 97.75 mass% or more.
<21> The method of producing a polycarbonate resin for a liquid crystal member according to the item <19> or

<20>, wherein the 3-pentadecylphenol has a purity of 99.33 mass% or more.

ADVANTAGEOUS EFFECTS OF INVENTION

The polycarbonate resin for a liquid crystal member according to one embodiment of the present invention is excellent in flowability and color tone, and is hence excellent in moldability and suitable for the production of, in particular, a liquid crystal member having a small thickness. In addition, the polycarbonate resin composition for a liquid crystal member containing the polycarbonate resin for a liquid crystal member according to the one embodiment of the present invention and an aromatic polycarbonate resin except the polycarbonate resin is also excellent in flowability and color tone, and is hence excellent in moldability and suitable for the production of, in particular, a liquid crystal member having a small thickness.

DESCRIPTION OF EMBODIMENTS

[Polycarbonate Resin for Liquid Crystal Member]

Apolycarbonate resin for a liquid crystal member of the present invention is obtained by using an end terminator containing 3-pentadecylphenol obtained from a natural product. The polycarbonate resin for a liquid crystal member of the present invention obtained by using the end terminator containing the 3-pentadecylphenol obtained from the natural product is hereinafter described.

<3-Pentadecylphenol obtained from Natural Product>

The end terminator containing the 3 -pentadecylphenol obtained from the natural product is used in the polycarbonate resin for a liquid crystal member of the present invention. Cardanol as an extract derived from a natural product such as a cashew nut shell liquid is used as the 3-pentadecylphenol obtained from the natural product. The cardanol in the cashew nut shell liquid is a mixture of mainly 3-pentadecylphenol, 3-pentadecylphenolmonoene, 3-pentadecylphenoldiene, and 3-pentadecylphenoltriene each represented by the following formula (III).

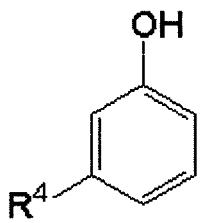

(III)

The formula (III) represents 3-pentadecylphenol in the case where $R^4$ represents $-(CH_2)_{14}CH_3$, represents 3-pentadecylphenolmonoene in the case where $R^4$ represents $-(CH_2)_7CH=CH(CH_2)_5CH_3$, represents 3-pentadecylphenoldiene in the case where $R^4$ represents $-(CH_2)_7CH=CHCH_2CH=CH(CH_2)CH_3$, and represents 3-pentadecylphenoltriene in the case where $R^4$ represents $-(CH_2)_7CH=CHCH_2CH=CHCH_2CH=CH_2$

As described above, the main component of the cardanol in the cashew nut shell liquid is formed of phenol derivatives each having, at the 3-position (meta position), a saturated hydrocarbon group having 15 carbon atoms or an unsaturated hydrocarbon group having 15 carbon atoms, the group having one to three double bonds.

In order to efficiently obtain the 3-pentadecylphenol using in the present invention, cardanol in the cashew nut shell liquid among the natural products is subj ected to a hydrogenation treatment; 3-pentadecylphenol thus obtained can be used as an end terminator upon production of the polycarbonate resin for a liquid crystal member of the present invention. In addition, the 3-pentadecylphenol obtained by subjecting the cardanol to the hydrogenation treatment contains about 7 mass% to 10 mass% of a resorcinol derivative and a phenol derivative except 3-pentadecylphenol as impurities, and when the resin is used as a liquid crystal member whose transparency is emphasized among the liquid crystal members, e.g., a light-guiding plate or a light-diffusing plate, the amount of the impurities is preferably reduced to the extent possible. The purity of the 3-pentadecylphenol is preferably 97.5 mass% or more, more preferably 97.75 mass% or more, still more preferably 99.33 mass% or more. It should be noted that the purity of the 3-pentadecylphenol is ideally 100 mass%.

A method for the hydrogenation reaction of the cardanol is not particularly limited and a typical hydrogenation method can be employed. A catalyst is, for example, a noble metal such as palladium, ruthenium, rhodium, or platinum, or nickel, or a product obtained by causing a carrier such as activated carbon, activated alumina, or diatomaceous earth to carry, on itself, a metal selected from those described above. A batch type involving performing the reaction while

suspending and stirring a powdery catalyst, or a continuous type involving using a reaction column filled with a molded catalyst can be adopted as a reaction system. At the time of the hydrogenation, a solvent may not be used depending on the type of the hydrogenation, but when the solvent is used, typical examples thereof include alcohols, ethers, esters, and saturated hydrocarbons. A reaction temperature at the time of the hydrogenation, which is not particularly limited, can be set to typically from 20°C to 250°C, preferably from 50°C to 200°C. When the reaction temperature is too low, a hydrogenation rate tends to reduce, and in contrast, when the reaction temperature is too high, the amount of a decomposition product tends to increase. A hydrogen pressure at the time of the hydrogenation can be set to typically from normal pressure to 80 kgf/cm$^2$ (from normal pressure to $78.4\times10^5$ Pa), preferably from 3 kgf/cm$^2$ to 50 kgf/cm$^2$ (from $2.9\times10^5$ Pa to $49.0\times10^5$ Pa).

The 3-pentadecylphenol obtained by the hydrogenation treatment method contains a resorcinol derivative and a phenol derivative except 3-pentadecylphenol as impurities. In order to increase the purity of the 3-pentadecylphenol by removing those impurities, for example, the following methods can be given: a method involving increasing the purity through distillation, a method involving increasing the purity through crystallization, and a method involving increasing the purity through distillation and crystallization after the distillation. Among them, the method involving performing the crystallization after the distillation is preferred.

For example, a method involving performing atmospheric distillation or vacuum distillation is known as the method involving increasing the purity through distillation, and the vacuum distillation is preferably employed. When the vacuum distillation is performed, it is preferred that the temperature and pressure of a main fraction be set to from 200°C to 260°C and from 1 mmHg to 10 mmHg, respectively, and a treatment be performed by using a filler in a vacuum distillation column. At this time, a reflux ratio (reflux amount/distillation amount) is preferably set to from 0.5 to 10. The following fillers may be used as the filler to be used in the vacuum distillation column: a Mc. MAHON packing, a Dixon packing, a Raschig ring, a Pall ring, a coil pack, a Heli pack, or the like, and among them, a Mc. MAHON packing is preferably used.

The method involving increasing the purity through crystallization is as describedbelow. The temperature of a solution prepared by dissolving the 3-pentadecylphenol containing the impurities in a crystallization solvent is reduced in a crystallization vessel, and 3-pentadecylphenol is precipitated by utilizing a difference between the supersaturated state of the 3-pentadecylphenol solution aiming at an increase in purity and the saturated concentration of the compound, whereby the crystal of 3-pentadecylphenol is produced. Next, the 3-pentadecylphenol in a crystalline state is subjected to solid-liquid separation from the solution. Thus, 3-pentadecylphenol increased in purity can be obtained. A crystallization operation can be performed in a wide temperature region ranging from the boiling point of the crystallization solvent to be used to itsmeltingpoint. Inaddition, the crystallization solvent is not particularly limited as long as the solvent can dissolve 3-pentadecylphenol, and acetone, ethyl acetate, a hydrocarbon-based solvent, acetonitrile, methanol, ethanol, or the like can be used. Among them, a hydrocarbon-based solvent can be given as a preferred solvent, and one or more of hexane and heptane can be given as more preferred solvents. It should be noted that when the temperature of the solution prepared by dissolving the 3-pentadecylphenol containing the impurities in the crystallization solvent is reduced in the crystallization vessel, the rate of the cooling can be appropriately set. The amount of the crystallization solvent can be appropriately set, but when the solvent is used in an amount of preferably from 2 parts by mass to 20 parts by mass, more preferably from 4 parts by mass to 10 parts by mass with respect to 1 part by mass of the 3-pentadecylphenol, efficient production can be achieved while a desired purity is secured. In addition, the crystallization can be performed without the addition of any seed crystal, but the crystallization can be efficiently performed by loading a seed crystal.

In addition, a controlled cooling method, a linear cooling method, a natural cooling method, or the like has been known as a method of reducing the temperature of the solution prepared by dissolving crude pentadecylphenol in the crystallization solvent in the crystallization vessel, but the method for the cooling is not particularly limited and the cooling rate can be appropriately set. Among them, a controlled cooling method is preferred because of the following reason. The degree of supersaturation of a saturated solution is kept low and constant all the time by reducing a temperature change (reducing the cooling rate) at an initial stage where the amount of the crystal is small and increasing the temperature change (increasing the cooling rate) at the final stage where the amount of the crystal increases, and hence the occurrence of a secondary nucleus is suppressed and only a monodisperse particle is obtained. The cooling rate is set to preferably from 0°C/h (constant temperature) to -10°C/h, more preferably from 0°C/h (constant temperature) to -5°C/h at the initial stage, and is set to preferably from -5°C/h to -30°C/h, more preferably from -10°C/h to -20°C/h at the final stage.

3-Pentadecylphenol obtained from a natural product and having a purity of preferably 97.5 mass% or more can be obtained from the crude pentadecylphenol by the purity-increasing method described above.

It is preferred that the content of a resorcinol derivative represented by the following general formula (I) in the 3-pentadecylphenol to be used in the present invention be 1 mass% or less and/or the content of a phenol derivative represented by the following general formula (II) therein be 2.5 mass% or less, and the total content of the resorcinol derivative and the phenol derivative therein be 2.5 mass% or less. In the case where the contents of the resorcinol

derivative and the phenol derivative deviate from the ranges, when the 3-pentadecylphenol is used as a raw material for a polymer material such as a polycarbonate resin, the transparency or appearance of the polymer material might be deteriorated. In addition, when the purity of high-purity 3-pentadecylphenol is 99.2 mass% or more, it is preferred that the content of the resorcinol derivative be 0.8 mass% or less and/or the content of the phenol derivative be 0.8 mass% or less, and the total content of the resorcinol derivative and the phenol derivative be 0.8 mass% or less.

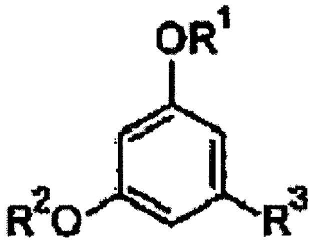
(I)
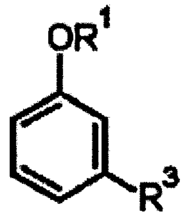
(II)

[In the general formulae (I) and (II), $R^1$ and $R^2$ each represent a hydrogen atom or an aliphatic hydrocarbon group having 1 to 20 carbon atoms, $R^3$ represents a hydrogen atom, or a saturated or unsaturated aliphatic hydrocarbon group having 1 to 20 carbon atoms, and $R^1$, $R^2$, and $R^3$ may be identical to or different from one another, provided that 3 -pentadecylphenol, which is represented by the general formula (II), where $R^1$ represents H and $R^3$ represents $C_{15}H_{31}$, is excluded.]

Examples of the aliphatic hydrocarbon group having 1 to 20 carbon atoms represented by $R^1$ or $R^2$ can include alkyl groups such as a methyl group, an ethyl group, a propyl group, a butyl group, a pentyl group, a hexyl group, a heptyl group, an octyl group, a nonyl group, a decyl group, a dodecyl group, a tetradecyl group, a pentadecyl group, a hexadecyl group, a heptadecyl group, and an octadecylgroup. Examples of the saturated or unsaturated aliphatic hydrocarbon group having 1 to 20 carbon atoms represented by $R^3$ can include, in addition to the alkyl groups given for $R^1$ and $R^2$, unsaturated aliphatic hydrocarbon groups serving as a monoene, diene, or triene having one or more unsaturated carbon-carbon double bonds in any one of the alkyl groups.

A compound in which $R^1$ and $R^2$ both represent a hydrogen atom as a compound included in the general formula (I) can be, for example, a compound in which $R^3$ represents an alkyl group having 1 to 20 carbon atoms such as 5-pentadecylresorcinol, 5-methylresorcinol, 5-ethylresorcinol, 5-propylresorcinol, 5-butylresorcinol, 5-hexylresorcinol, 5-octylresorcinol, 5-decylresorcinol, 5-dodecylresorcinol, 5-tetradecylresorcinol, 5-octadecylresorcinol, or 5-nonyldecylresorcinol, and may be a compound having an unsaturated aliphatic hydrocarbon group such as a monoene, diene, or triene having one or more unsaturated carbon-carbon double bonds in the alkyl group.

In addition, a compound represented by the general formula (I), where: $R^1$ represents an aliphatic hydrocarbon group having 1 to 20 carbon atoms; $R^2$ represents a hydrogen atom; and $R^3$ represents a saturated or unsaturated aliphatic hydrocarbon group having 1 to 20 carbon atoms, can be, for example, a compound such as 3-methoxy-5-pentadecylphenol, 3-ethoxy-5-pentadecylphenol, 3-propoxy-5-pentadecylphenol, 3-butoxy-5-pentadecylphenol, 3-methoxy-5-hexylphenol, 3-methoxy-5-octylphenol, 3-methoxy-5-decylphenol, 3-methoxy-5-dodecylphenol, 3-methoxy-5-tetradecylphenol, 3-methoxy-5-heptadecylphenol, 3-methoxy-5-octadecylphenol, 3-methoxy-5-nonyldecylphenol, 3-ethoxy-5-hexylphenol, 3-ethoxy-5-octylphenol, 3-ethoxy-5-decylphenol, 3-ethoxy-5-dodecylphenol, 3-ethoxy-5-tetradecylphenol, 3-ethoxy-5-heptadecylphenol, 3-ethoxy-5-octadecylphenol, or 3-ethoxy-5-nonyldecylphenol, and may be a compound having an unsaturated aliphatic hydrocarbon group such as a monoene, diene, or triene having one or more unsaturated carbon-carbon double bonds in the alkyl group at its 5-position.

A compound in which $R^1$ represents hydrogen and $R^3$ represents a saturated or unsaturated aliphatic hydrocarbon group having 1 to 20 carbon atoms as a compound included in the general formula (II) may be a compound having an alkyl group at its 3-position such as 3-hexylphenol, 3-octylphenol, 3-decylphenol, 3-dodecylphenol, 3-tridecylphenol, 3-tetradecylphenol, 3-hexadecylphenol, 3-octadecylphenol, or 3-nonyldecylphenol, or a compound having an unsaturated aliphatic hydrocarbon group such as a monoene, diene, or triene having one or more unsaturated carbon-carbon double bonds in the alkyl group.

In addition, a compound in which $R^1$ represents an aliphatic hydrocarbon group having 1 to 20 carbon atoms can be, for example: a compound such as 1-methoxy-3-hexylbenzene, 1-ethoxy-3-hexylbenzene, 1-propoxy-3-hexylbenzene, 1-butoxy-3-hexylbenzene, 1-pentoxy-3-hexylbenzene, 1-hexoxy-3-hexylbenzene, 1-octoxy-3-hexylbenzene, 1-decoxy-3-hexylbenzene, 1-dodecoxy-3-hexylbenzene, or 1-butyrodetoxy-3-hexylbenzene when the compound has a hexyl group at its 3-position; or a compound such as 1-methoxy-3-pentadecylbenzene, 1-ethoxy-3-pentadecylbenzene, 1-propoxy-3-pentadecylbenzene, 1-butoxy-3-pentadecylbenzene, 1-pentoxy-3-pentadecylbenzene, 1-hexoxy-3-pentadecylbenzene, 1-octoxy-3-pentadecylbenzene, 1-decoxy-3-pentadecylbenzene, 1-dodecoxy-3-pentadecylbenzene, or 1-butyrodetoxy-3-pentadecylbenzene when the compound has a pentadecyl group at its 3-position.

It should be noted that the alkyl groups given for the general formula (I) and the general formula (II) may be linear alkyl groups or may be branched alkyl groups.

<Method of producing Polycarbonate Resin for Liquid Crystal Member>

Next, a method of producing the polycarbonate resin for a liquid crystal member of the present invention is described. In order to produce the polycarbonate resin for a liquid crystal member of the present invention, the end terminator containing the 3-pentadecylphenol obtained from the natural product serving as an end group needs to be used. In particular, an end terminator containing 3-pentadecylphenol obtained by performing distillation and crystallization as described above is preferably used. An end terminator for producing a polycarbonate resin that has heretofore been used can be used as an end terminator except the 3-pentadecylphenol obtained from the natural product (other end terminator), and examples thereof include phenol, p-cresol, p-t-butylphenol, p-cumylphenol, tribromophenol, nonylphenol, and p-t-octylphenol. Such other end terminator may be used in combination with the 3-pentadecylphenol obtained from the natural product, and the other end terminator to be used in combination therewith is particularly preferably p-t-butylphenol or p-cumylphenol. When the 3-pentadecylphenol obtained from the natural product and the other end terminator are used in combination, a usage ratio between the terminators " (3-pentadecylphenol) : (other end terminator) " is preferably from 99:1 to 20:80, more preferably from 90:10 to 30:70 in terms of a molar ratio.

In addition, the composition amount of an end group derived from OH with respect to all end groups in the polycarbonate resin is preferably 5.0 mol% or less, more preferably 3.0 mol% or less, particularly preferably 1.0 mol% or less. When the composition amount of the end group derived from OH falls within the range, the polycarbonate resin to be obtained shows additionally high heat stability.

In order to produce the polycarbonate resin for a liquid crystal member of the present invention, a dihydric phenol for constituting its main chain needs to be used. Any one of the various known dihydric phenols can be used as the dihydric phenol, but a dihydric phenol represented by the following general formula (1) is preferably used.

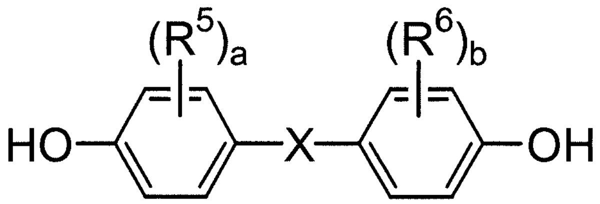

(1)

Here, in the general formula (1), $R^5$ and $R^6$ each independently represent an alkyl group or alkoxy group having 1 to 6 carbon atoms, X represents a single bond, an alkylene group having 1 to 8 carbon atoms, an alkylidene group having 2 to 8 carbon atoms, a cycloalkylene group having 5 to 15 carbon atoms, a cycloalkylidene group having 5 to 15 carbon atoms, -S-, -SO-, $-SO_2-$, -O-, or -CO-, and a and b each represent an integer of from 0 to 4.

The dihydric phenol represented by the general formula (1) is not particularly limited, but 2,2-bis(4-hydroxyphenyl)propane [trivial name: bisphenol A] is suitable.

Examples of the dihydric phenol except bisphenol A include:

bis(hydroxyaryl)alkanes such as bis(4-hydroxyphenyl)methane, 1,1-bis(4-hydroxyphenyl)ethane, 2,2-bis(4-hydroxyphenyl)butane, 2,2-bis(4-hydroxyphenyl)octane, bis(4-hydroxyphenyl)phenylmethane, bis(4-hydroxyphenyl)diphenylmethane, 2,2-bis(4-hydroxy-3-methylphenyl)propane, bis(4-hydroxyphenyl)naphthylmethane, 1,1-bis(4-hydroxy-t-butylphenyl)propane, 2,2-bis(4-hydroxy-3-bromophenyl)propane, 2,2-bis(4-hydroxy-3,5-dimethylphenyl)propane, 2,2-bis(4-hydroxy-3-chlorophenyl)propane, 2,2-bis(4-hydroxy-3,5-dichlorophenyl)propane, and 2,2-bis(4-hydroxy-3,5-dibromophenyl)propane;

bis(hydroxyaryl)cycloalkanes such as 1,1-bis(4-hydroxyphenyl)cyclopentane, 1,1-bis(4-hydroxyphenyl)cyclohexane, 1,1-bis(4-hydroxyphenyl)-3,5,5-trimethylcyclohexane, 2,2-bis(4-hydroxyphenyl)norbornane, and 1,1-bis(4-hydroxyphenyl)cyclododecane; dihydroxyaryl ethers such as 4,4'-dihydroxydiphenyl ether and 4,4'-dihydroxy-3,3'-dimethylphenyl ether; dihydroxydiaryl sulfides such as 4,4'-dihydroxydiphenyl sulfide and 4,4'-dihydroxy-3,3'-dimethyldiphenyl sulfide; dihydroxydiaryl sulfoxides such as 4,4'-dihydroxydiphenyl sulfoxide and 4,4'-dihydroxy-3,3'-dimethyldiphenyl sulfoxide; dihydroxydiaryl sulfones such as 4,4'-dihydroxydiphenyl sulfone and 4,4'-dihydroxy-3,3'-dimethyldiphenyl sulfone; dihydroxydiphenyls such as 4,4'-dihydroxydiphenyl; dihydroxydiarylfluorenes such as 9,9-bis(4-hydroxyphenyl)fluorene and 9,9-bis(4-hydroxy-3-methylphenyl)fluorene; dihydroxydiaryladamantanes such as 1,3-bis(4-hydroxyphenyl)adamantane, 2,2-bis(4-hydroxyphenyl)adamantane, and 1,3-bis(4-hydroxyphenyl)-5,7-dimethyladamantane;

4,4'-[1,3-phenylenebis(1-methylethylidene)]bisphenol;

10,10-bis(4-hydroxyphenyl)-9-anthrone; and

1,5-bis(4-hydroxyphenylthio)-2,3-dioxapentane.

Each of those dihydric phenols may be used alone, or two or more thereof may be used as a mixture.

Further, a dihydric phenol containing a constituent unit represented by the following formula (2) can be used as a dihydric phenol not included in the dihydric phenol represented by the general formula (1) in combination with the dihydric phenol represented by the general formula (1). The use of a copolymer having such constituent unit can improve the flame retardancy of the polycarbonate resin for a liquid crystal member of the present invention to be obtained. The dihydric phenol containing a constituent unit represented by the following general formula (2) is represented by a polyorganosiloxane represented by the following general formula (2-1).

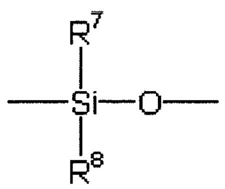 (2) 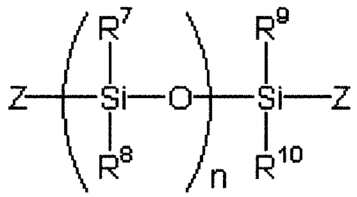 (2-1)

In the general formula (2) or the general formula (2-1), $R^7$, $R^8$, $R^9$, and $R^{10}$ each independently represent a hydrogen atom, a halogen atom, an alkyl group having 1 to 6 carbon atoms, an alkoxy group having 1 to 6 carbon atoms, or an aryl group having 6 to 12 carbon atoms, Z represents a phenol residue having a trimethylene group derived from a phenol compound having an allyl group, and n represents from 70 to 1,000.

The polyorganosiloxane represented by the general formula (2-1) is obtained by modifying an end of a polyorganosiloxane having hydrogen at the end with a phenol compound having an allyl group such as 2-allylphenol or eugenol. The polyorganosiloxane having an end modified with the phenol compound having an allyl group can be synthesized by a method disclosed in JP 2662310 B2.

Polydimethylsiloxane is suitable as the polyorganosiloxane.

Further, a branching agent can be added to the dihydric phenol so that the polycarbonate resin may have a branched structure in the main chain thereof. The addition amount of the branching agent is preferably from 0.01 mol% to 3 mol%, more preferably from 0.1 mol% to 1.0 mol% with respect to the dihydric phenol.

Examples of the branching agent include compounds each having three or more functional groups such as 1,1,1-tris(4-hydroxyphenyl)ethane, 4,4'-[1-[4-[1-(4-hydroxyphenyl)-1-methylethyl]phenyl]ethyliden e]bisphenol, α,α',α"-tris(4-hydroxyphenyl)-1,3,5-triisopropylbenzene, 1-[α-methyl-α-(4'-hydroxyphenyl)ethyl]-4-[α',α'-bis(4"-hydroxyphenyl)ethyl]benzene, phloroglucin, trimellitic acid, and isatinbis(o-cresol).

The polycarbonate resin of the present invention is produced by causing a carbonate raw material and the dihydric phenol to react with each other. The carbonate raw material refers to a compound capable of producing a carbonate bond in a polycarbonate main chain through a polymer-producing reaction such as a condensation reaction or an exchange reaction. Examples of such compound in the case where the polycarbonate is produced by an interfacial polycondensation method include phosgene, triphosgene, bromophosgene, bis(2,4,6-trichlorophenyl) carbonate, bis(2,4-dichlorophenyl) carbonate, bis(2-cyanophenyl) carbonate, and trichloromethyl chloroformate.

In addition, in the production of a polycarbonate by an ester exchange reaction method (melting method), a carbonic acid diester is used as the carbonate raw material, and examples of the carbonic acid diester include a diaryl carbonate compound, a dialkyl carbonate compound, and an alkyl aryl carbonate compound.

Herein, specific examples of the diaryl carbonate compound include diphenyl carbonate, ditolyl carbonate, bis(chlorophenyl) carbonate, m-cresyl carbonate, dinaphthyl carbonate, bis (diphenyl) carbonate, and bisphenol A bisphenyl carbonate. Specific examples of the dialkyl carbonate compound include diethyl carbonate, dimethyl carbonate, dibutyl carbonate, dicyclohexyl carbonate, and bisphenol A bismethyl carbonate. Specific examples of the alkyl aryl carbonate compound include methyl phenyl carbonate, ethyl phenyl carbonate, butyl phenyl carbonate, cyclohexyl phenyl carbonate, and bisphenol A methyl phenyl carbonate.

The polycarbonate resin for a liquid crystal member of the present invention can be produced by employing a method commonly employed in typical production of a polycarbonate, e.g., an interfacial polycondensation method involving using phosgene or a phosgene derivative, or the ester exchange method (melting method). Among them, an interfacial polycondensation method is preferred. Examples of the interfacial polycondensation method involving using phosgene or the phosgene derivative include: a method involving synthesizing a polycarbonate oligomer of the dihydric phenol from the dihydric phenol and phosgene or the phosgene derivative in advance, adding an alkali aqueous solution containing the dihydric phenol and the end terminator containing the 3-pentadecylphenol obtained from the natural product to a solution of the oligomer in an inert organic solvent, and subjecting the mixture to a reaction; and a method involving adding phosgene or the phosgene derivative to a mixed liquid of the alkali aqueous solution of the dihydric phenol, the end terminator containing the 3-pentadecylphenol obtained from the natural product, and the inert organic solvent, and subjecting the mixture to a reaction. Among them, the former method, i.e., an oligomer method is suitable.

Next, a method of producing the polycarbonate resin for a liquid crystal member of the present invention by the oligomer method is described. First, the alkali aqueous solution of the dihydric phenol (aqueous solution of sodium hydroxide or the like) is prepared by dissolving the dihydric phenol in an aqueous solution of an alkali metal hydroxide. Next, the polycarbonate oligomer of the dihydric phenol is synthesized by introducing phosgene or the phosgene derivative into a mixed liquid of the alkali aqueous solution and the inert organic solvent (organic solvent such as methylene chloride). At this time, the alkali concentration of the alkali aqueous solution preferably falls within the range of from 1 mass% to 15 mass%, and a volume ratio between an organic phase and an aqueous phase desirably falls within the range of from 5:1 to 1:7, preferably from 2:1 to 1:4. A reaction temperature is adjusted by cooling with a water bath and is selected from the range of typically from 0°C to 50°C, preferably from 5°C to 40°C, and a reaction time is from about 15 minutes to 4 hours, preferably from about 30 minutes to 2 hours. The degree of polymerization of the polycarbonate oligomer thus obtained is typically 20 or less, preferably from about 2 to 10.

Next, the alkali aqueous solution of the dihydric phenol and the end terminator containing the 3-pentadecylphenol obtained from the natural product, and as desired, the inert organic solvent are added to the organic phase containing the polycarbonate oligomer thus obtained, and the contents are subjected to interfacial polycondensation at a temperature in the range of typically from 0°C to 50°C, preferably from 5°C to 40°C for from about 10 minutes to 6 hours by performing stirring or the like to bring the contents into contact with one another. At this time, the alkali concentration of the alkali aqueous solution is preferably from 1 mass% to 15 mass%, and a volume ratio between the organic phase and the aqueous phase desirably falls within the range of from 7:1 to 1: 2, preferably from 4:1 to 1:1. In addition, a ratio between the dihydric phenol and the polycarbonate oligomer is selected so that a molar ratio " (dihydric phenol)/(chloroformate group of the polycarbonate oligomer) " may be typically from 0.4 to 0.55, preferably from 0.45 to 0.5. In addition, a ratio between the alkali metal hydroxide and the polycarbonate oligomer is selected so that a molar ratio " (alkali metal hydroxide) / (chloroformate group of the polycarbonate oligomer) " may be typically from 1.0 to 2.0, preferably from 1.2 to 1.7. In addition, the usage amount of the end terminator is selected so that a molar ratio "(end terminator)/(chloroformate group of the polycarbonate oligomer)" may be typically from 0.02 to 0.20, preferably from 0.04 to 0.17. Further, in the reaction, a catalyst can be used as desired. The usage amount of the catalyst is selected so that a molar ratio " (catalyst) / (chloroformate group of the polycarbonate oligomer) " may be typically from $1.0\times10^{-3}$ to $10.0\times10^{-3}$, preferably from $1.0\times10^{-3}$ to $5.0\times10^{-3}$.

Examples of the alkali metal hydroxide to be used in the production of the polycarbonate resin for a liquid crystal member of the present invention include sodium hydroxide, potassium hydroxide, lithium hydroxide, and cesium hydroxide. Among them, sodium hydroxide and potassium hydroxide are suitable. In addition, the inert organic solvent comes in various kinds. Examples thereof include chlorinated hydrocarbons such as: dichloromethane (methylene chloride); chloroform; 1,1-dichloroethane; 1,2-dichloroethane; 1,1,1-trichloroethane; 1,1,2-trichloroethane; 1,1,1,2-tetrachloroethane; 1,1,2,2-tetrachloroethane; pentachloroethane; and chlorobenzene, and acetophenone. One of those organic solvents may be used alone, or two or more thereof may be used in combination. Among them, chloroform or methylene chloride is preferred, and methylene chloride is particularly suitable.

Any one of the various catalysts can be used as the catalyst. A quaternary ammonium salt, a quaternary phosphonium salt, a tertiary amine, or the like is specif icallyused, and examples of the quaternary ammonium salt include trimethylbenzylammonium chloride, triethylbenzylammonium chloride, tributylbenzylammonium chloride, trioctylmethylammonium chloride, tetrabutylammonium chloride, and tetrabutylammonium bromide. In addition, examples of the quaternary phosphonium salt include tetrabutylphosphonium chloride and tetrabutylphosphonium bromide. In addition, examples of the tertiary amine include triethylamine, tributylamine, N,N-dimethylcyclohexylamine, pyridine, and dimethylaniline.

Among the catalysts, a tertiary amine is preferred, and triethylamine is particularly suitable.

An operation of recovering the polycarbonate resin thus obtained from the organic solvent solution containing the resin is performed in accordance with a typical method. Thus, the polycarbonate resin for a liquid crystal member of the present invention can be obtained.

In the production of the polycarbonate by the ester exchange reaction method (melting method), the dihydric phenol, the carbonic acid diester, and the end terminator containing the 3-pentadecylphenol, and as required, a branching agent or the like are subjected to an ester exchange reaction in a molten state, and phenol to be produced as a by-product is removed to the outside of a system under a reduced pressure condition or the like, whereby the polycarbonate resin can be obtained. In the ester exchange reaction method, an ester exchange catalyst can be used for accelerating the reaction. Preferred examples of the ester exchange catalyst include salts of sodium, calcium, and cesium, ammonium salts, and phosphonium salts.

The polycarbonate resin for a liquid crystal member of the present invention is obtained by using an end terminator containing 3-pentadecylphenol obtained from a natural product, and its viscosity-average molecular weight, which is not particularly limited, is desirably set to from 8,000 to 30,000, preferably from 8, 000 to 22, 000, more preferably from 8, 000 to 19, 000, particularly preferably from 8,000 to 14,000 from the viewpoint of maintaining flowability and strength upon molding into a thin liquid crystal member.

The polycarbonate resin for a liquid crystal member of the present invention is excellent in color tone and has a YI value in the following measurement method of preferably 1.1 or less, more preferably 1.0 or less. In addition, the resin has a light transmittance at a wavelength of 400 nm in the following measurement method of preferably 85% or more, more preferably 87% or more, still more preferably 88.1% or more.

<Method of measuring YI Value>

500 ppm by mass of bis(2,6-di-t-butyl-4-methylphenyl)pentaerythritol phosphite is added to the polycarbonate resin, the mixture is melted, kneaded, and extruded with a vented single screw extruder of 40 mmφ at a resin temperature of 280°C and a screw rotation speed of 100 rpm to provide a pellet, the resultant pellet is molded into a molded article having a thickness of 3 mm, and the molded article is subjected to measurement with a spectrophotometer by a transmission method at a measurement area of 30 φ under a C2 light source.

<Method of measuring Light Transmittance at Wavelength of 400 nm>

500 ppm by mass of bis(2,6-di-t-butyl-4-methylphenyl)pentaerythritol phosphite is added to the polycarbonate resin, the mixture is melted, kneaded, and extruded with a vented single screw extruder of 40 mmφ at a resin temperature of 280°C and a screw rotation speed of 100 rpm to provide a pellet, the resultant pellet is molded into a molded article having a thickness of 3 mm, and the molded article is subjected to measurement of a total light transmittance with a spectral photometer.

When the polycarbonate resin for a liquid crystal member of the present invention is mixed with an aromatic polycarbonate resin except the polycarbonate resin for a liquid crystal member at an arbitrary ratio, a polycarbonate resin composition for a liquid crystal member can be obtained. An additive such as an antioxidant, a UV absorber, a flame retardant, a release agent, an inorganic filler (e.g., aglassfiber, talc, titaniumoxide, ormica), acolorant, or a light-diffusing agent can be used in the polycarbonate resin for a liquid crystal member of the present invention or the polycarbonate resin composition for a liquid crystal member as required depending on characteristics which a target liquid crystal member is required to have.

The polycarbonate resin for a liquid crystal member or the polycarbonate resin composition for a liquid crystal member containing the polycarbonate resin for a liquid crystal member and the aromatic polycarbonate resin except the polycarbonate resin for a liquid crystal member can be molded into a liquid crystal member for a liquid crystal display apparatus to be used in, for example, a cellular phone, a liquid crystal television, a personal computer, an electronic dictionary, or an electronic book by any one of the various molding methods such as injection molding, injection compression molding, extrusion molding, and blow molding.

The polycarbonate resin for a liquid crystal member of the present invention and the polycarbonate resin composition for a liquid crystal member using the resin are each excellent in flowability and color tone. Accordingly, in particular, when a molded body having a small thickness is produced, the resin or the composition is desirably molded by injection molding, and can be suitably used as a resin for a light-guiding plate or light-diffusing plate for a liquid crystal display apparatus.

EXAMPLES

The present invention is hereinafter described more specif ically by way of Examples and Comparative Examples. It should be noted that the present invention is not limited by these examples. It should be noted that measurements and evaluations in Examples and Comparative Examples were performed by the following methods.

<Measurement of Viscosity-average Molecular Weight (Mv)>

A viscosity-average molecular weight (Mv) is calculated from the following equation by using a limiting viscosity [η] determined by measuring the viscosity of a methylene chloride solution at 20°C with an Ubbelohde-type viscometer.

$$[\eta]=1.23\times10^{-5}Mv^{0.83}$$

<Methods of measuring Purity and Impurity Amount of 3-Pentadecylphenol>

The amount of each of 3-pentadecylphenol and a resorcinol derivative was measured by means of liquid chromatography (manufactured by Agilent Technologies, product name: "AGILENT 1200") through the use of "L-column

ODS" (manufactured by Chemicals Evaluation and Research Institute, Japan, 4.6 mmID×150 mm, particle diameter: 3 μm) as a column and a buffer containing acetonitrile and formic acid at a ratio of 95/5 (vol/vol) as a mobile phase.

The amount of a phenol derivative was measured with a gas chromatograph mass spectrometer (manufactured by JEOL Ltd., product name: "JMS-Q1000GC") and a column "VF-1" measuring 30 m in length by 250 μm in inner diameter by 0.25 μm in thickness.

<Measurement of End Group Composition Amount>

The end group composition amount of a polycarbonate resin was calculated by measuring its $^1$H-NMR spectrum with an NMR apparatus (manufactured by JEOL Ltd., product name: "JNM-LA500").

<Amount of Unreacted PDP>

2 g of a polycarbonate resin pellet was dissolved in 15 ml of chloroform, and 25 ml of hexane was added to the solution to precipitate a polycarbonate resin. After that, 20 ml of the supernatant was collected, and was concentrated to dryness, followed by the addition of 10 ml of a mixed solvent obtained by mixing THF, water, and acetonitrile at a volume ratio of 9/7/14. The amount of a PDP in the mixed solution was determined by means of high performance liquid chromatography (manufactured by JASCO Corporation, product name: "LC-2000"), and the amount of an unreacted PDP in the polycarbonate resin was calculated.

<Measurement of Flow Value (Q Value)>

The amount ($\times 10^{-2}$ mL/sec) of a molten resin flowing out of a nozzle having a diameter of 1 mm and a length of 10 mm was measured with a Koka flow tester according to JIS K 7210 at 280°C under a pressure of 15.7 MPa.

<Evaluation for Thin-wall Moldability>

500 ppm by mass of ADEKASTAB PEP36 [manufactured by ADEKA Corporation, bis(2,6-di-t-butyl-4- methylphenyl)pentaerythritol phosphite] was added to each of polycarbonate resins obtained in Reference Examples 1 to 12, Examples 1 and 2, and Comparative Examples 1 to 3, and the mixture was melted, kneaded, and extruded with a vented single screw extruder of 40 mmφ at a resin temperature of 280°C and a screw rotation speed of 100 rpm to provide a pellet. Each of the resultant pellets was molded into a flat plate measuring 50 mm by 90 mm by 0.4 mm, and the moldability of the pellet was evaluated as AA, A, B, or X by the following evaluation criteria.

AA: The resin was able to be loaded into 100% of the area of the flat plate having a thickness of 0.4 mm and was able to be molded into the flat plate.
A: The resin was able to be loaded into from 75% to less than 100% of the area of the flat plate having a thickness of 0.4 mm.
B: The resin was able to be loaded into only from 50% to less than 75% of the area of the flat plate having a thickness of 0.4 mm.
X: The resin was able to be loaded into only less than 50% of the area of the flat plate having a thickness of 0.4 mm.

<Method of measuring YI Value>

500 ppm by mass of ADEKASTAB PEP36 [manufactured by ADEKA Corporation, bis(2,6-di-t-butyl-4- methylphenyl)pentaerythritol phosphite] was added to each of polycarbonate resins obtained in Reference Examples 1 to 12, Examples 1 and 2, and Comparative Examples 1 to 3, and the mixture was melted, kneaded, and extruded with a vented single screw extruder of 40 mmφ at a resin temperature of 280°C and a screw rotation speed of 100 rpm to provide a pellet. The resultant pellet was molded into a molded article having a thickness of 3 mm at 320°C, and the molded article was subjected to measurement with a spectrophotometer (manufactured by Nippon Denshoku Industries Co. , Ltd, product name: "Σ90") by a transmission method at a measurement area of 30 φ under a C2 light source.

<Method of measuring Light Transmittance at Wavelength of 400 nm>

500 ppm by mass of ADEKASTAB PEP36 [manufactured by ADEKA Corporation, bis(2,6-di-t-butyl-4- methylphenyl)pentaerythritol phosphite] was added to each of polycarbonate resins obtained in Reference Examples 1 to 12 , Examples 1 and 2, and Comparative Examples 1 to 3, and the mixture was melted, kneaded, and extruded with a vented single screw extruder of 40 mmφ at a resin temperature of 280°C and a screw rotation speed of 100 rpm to provide a

pellet. The resultant pellet was molded into a molded article having a thickness of 3 mm at 320°C, and the molded article was subjected to measurement of a total light transmittance with a spectral photometer (manufactured by Hitachi High-Technologies Corporation, product name: "U-4100").

Reference Example 1

(1) Production of Polycarbonate Oligomer

0.2 mass% of sodium dithionite with respect to bisphenol A (BPA) to be dissolved later was added to aqueous sodium hydroxide having a concentration of 5.6 mass%, and BPA was dissolved in the mixture so that a BPA concentration became 13.5 mass%. Thus, a solution of BPA in aqueous sodium hydroxide was prepared. The solution of BPA in aqueous sodium hydroxide and methylene chloride were continuously passed through a tubular reactor having an inner diameter of 6 mm and a tube length of 30 m at flow rates of 40 L/hr and 15 L/hr, respectively, and at the same time, phosgene was continuously passed therethrough at a flow rate of 4.0 kg/hr. The tubular reactor had a jacket portion and the temperature of a reaction liquid was kept at 40°C or less by passing cooling water through the jacket.

The reaction liquid delivered from the tubular reactor was continuously introduced into a baffled vessel-type reactor mounted with a sweptback blade and having an internal volume of 40 L. The solution of BPA in aqueous sodium hydroxide, 25 mass% aqueous sodium hydroxide, water, a 1 mass% aqueous solution of triethylamine, and a 20 mass% solution of p-t-butylphenol (PTBP) in methylene chloride were further supplied to the reactor at flow rates of 2.8 L/hr, 0.07 L/hr, 17 L/hr, 0.64 L/hr, and 149.2 kg/hr, respectively, and the mixture was subjected to a reaction at from 29°C to 32°C. A reaction liquid was continuously taken out from the vessel-type reactor, and an aqueous phase was separated and removed by leaving the reaction liquid at rest, followed by the collection of a methylene chloride phase. A polycarbonate oligomer solution thus obtained had an oligomer concentration of 315 g/L and a chloroformate group concentration of 0.75 mol/L.

(2) Production of Polycarbonate Resin for Liquid Crystal Member

333 mL of the polycarbonate oligomer solution and 217 mL of methylene chloride were loaded into a vessel-type reactor mounted with a baffle board and a paddle-type stirring blade, and having an internal volume of 1 L, and 14 g of 3-pentadecylphenol (m-PDP) [manufactured by Tokyo Chemical Industry Co., Ltd., purity: 92.10 mass%, resorcinol derivative: 2.15 mass%, phenol derivative: 5.11 mass%] was dissolved in the mixture, followed by the addition of 111 μL of triethylamine to the solution. 33.3 g of 6.4 mass% aqueous sodium hydroxide was added to the mixture under stirring, and the whole was subjected to a reaction for 10 minutes. Next, a solution of BPA in aqueous sodium hydroxide (prepared by dissolving 27.4 g of BPA in an aqueous solution prepared by dissolving 14 g of NaOH and 55 mg of sodium dithionite in 203 mL of water) was added to the resultant, and the mixture was subjected to a polymerization reaction for 50 minutes.

200 mL of methylene chloride was added to the resultant for dilution, and the mixture was stirred for 10 minutes. After that, the mixture was separated into an organic phase containing a polycarbonate resin, and an aqueous phase containing excess amounts of bisphenol A and NaOH, and the organic phase was isolated. The resultant solution of the polycarbonate resin in methylene chloride was sequentially washed with 15 vol% each of 0.03 mol/L aqueous NaOH and 0.2 mol/L hydrochloric acid with respect to the solution, and was then repeatedly washed with pure water until an electric conductivity in the aqueous phase after the washing became 0.05 μS/m or less. The solution of the polycarbonate resin in methylene chloride obtained by the washing was concentrated and pulverized, and the resultant flake was dried under reduced pressure at 100°C to provide the polycarbonate resin. The end composition of the resultant polycarbonate resin derived from m-PDP and PTBP used as end terminators was as follows: the polycarbonate resin had an end group derived from m-PDP at 6.87 mol% and an end group derived from PTBP at 1.44 mol%. In addition, the resultant polycarbonate resin had a viscosity-average molecular weight (Mv) of 8,700 and a flow value (Q value) of $165\times10^{-2}$ mL/sec, and its thin-wall moldability was evaluated as AA. The results of those measurements are shown in Table 1.

Reference Example 2

The same operations as those of Reference Example 1 were performed except that in the section (2) of Reference Example 1, the amount of m-PDP was changed to 8.8 g. The results of the measurements are shown in Table 1.

Reference Example 3

The same operations as those of Reference Example 1 were performed except that in the section (2) of Reference Example 1, the amount of m-PDP was changed to 7.6 g. The results of the measurements are shown in Table 1.

Reference Example 4

The same operations as those of Reference Example 1 were performed except that in the section (2) of Reference Example 1, the amounts of the oligomer solution, methylene chloride, m-PDP, triethylamine, and 6.4 mass% aqueous sodium hydroxide were changed to 349 mL, 191 mL, 1. 6 g, 110 μL, and 33 g, respectively, the solution of BPA in aqueous sodium hydroxide was changed to a solution prepared by dissolving 26.9 g of BPA in an aqueous solution prepared by dissolving 14 g of NaOH and 54 mg of sodium dithionite in 199 mL of water, and a solution prepared by dissolving 2.4 g of PTBP in 10 mL of methylene chloride was added simultaneously with the addition of the solution of BPA in aqueous sodium hydroxide. The results of the measurements are shown in Table 1.

Reference Example 5

The same operations as those of Reference Example 3 were performed except that in Reference Example 3, the amounts of m-PDP and PTBP were changed to 3.2 g and 1. 6 g, respectively. The results of the measurements are shown in Table 1.

Reference Example 6

The same operations as those of Reference Example 5 were performed except that in Reference Example 5, the amounts of m-PDP and PTBP were changed to 4.9 g and 0. 8 g, respectively. The results of the measurements are shown in Table 1.

Reference Example 7

The same operations as those of Reference Example 1 were performed except that in the section (2) of Reference Example 1, the amounts of the polycarbonate oligomer solution, methylene chloride, m-PDP, triethylamine, and 6.4 mass% aqueous sodium hydroxide were changed to 13.4 L, 9.8 L, 280 g, 4.1 mL, and 85 g, respectively, and the solution of BPA in aqueous sodium hydroxide was changed to a solution prepared by dissolving 1,176 g of BPA in an aqueous solution prepared by dissolving 545 g of NaOH and 2.2 g of sodium dithionite in 8 L of water. The results of the measurements are shown in Table 1.

Reference Example 8

The same operations as those of Reference Example 1 were performed except that in the section (2) of Reference Example 1, the amounts of the polycarbonate oligomer solution, methylene chloride, m-PDP, triethylamine, and 6.4 mass% aqueous sodium hydroxide were changed to 143 mL, 82 mL, 2 g, 2 μL, and 26.8 g, respectively, and the solution of BPA in aqueous sodium hydroxide was changed to a solution prepared by dissolving 9.8 g of BPA in an aqueous solution prepared by dissolving 4.7 g of NaOH and 20 mg of sodium dithionite in 69 mL of water. The results of the measurements are shown in Table 1.

Reference Example 9

The same operations as those of Reference Example 1 were performed except that in the section (2) of Reference Example 1, the amounts of the polycarbonate oligomer solution, methylene chloride, m-PDP, and triethylamine were changed to 286 mL, 164 mL, 4 g, and 90 μL, respectively, and polymerization was performed for 60 minutes by adding a solution of BPA in aqueous sodium hydroxide (solution prepared by dissolving 22 g of BPA in an aqueous solution prepared by dissolving 12.9 g of NaOH and 44 mg of sodium dithionite in 188 mL of water) instead of 6.4 mass% aqueous sodium hydroxide. The results of the measurements are shown in Table 1.

Reference Example 10

(1) Production of Polycarbonate Oligomer

0.2 mass% of sodium dithionite with respect to BPA to be dissolved later was added to aqueous sodium hydroxide having a concentration of 5.6 mass%, and BPA was dissolved in the mixture so that a BPA concentration became 13.5 mass%. Thus, a solution of the monomer in aqueous sodium hydroxide was prepared. The solution of the monomer in aqueous sodium hydroxide and methylene chloride were continuously passed through a tubular reactor having an inner diameter of 6 mm and a tube length of 30 m at flow rates of 40 L/hr and 35 L/hr, respectively, and at the

same time, phosgene was continuously passed therethrough at a flow rate of 4.0 kg/hr. The tubular reactor had a jacket portion and the temperature of a reaction liquid was kept at 40°C or less by passing cooling water through the jacket.

An aqueous phase was separated and removed by leaving the reaction liquid delivered from the tubular reactor at rest, and a methylene chloride phase was collected. A polycarbonate oligomer solution thus obtained had an oligomer concentration of 225 g/L and a chloroformate group concentration of 0.73 mol/L.

(2) Production of Polycarbonate Resin for Liquid Crystal Member

489 mL of the oligomer solution and 61 mL of methylene chloride were loaded into a vessel-type reactor mounted with a baffle board and a paddle-type stirring blade, and having an internal volume of 1 L, and 9.6 g of 3-pentadecylphenol (m-PDP) [manufactured by Tokyo Chemical Industry Co., Ltd. , purity: 92.10 mass%] was dissolved in the mixture, followed by the addition of 149 μL of triethylamine to the solution. 47 g of 6.4 mass% aqueous sodium hydroxide was added to the mixture under stirring, and the whole was subjected to a reaction for 10 minutes. Next, a solution of BPA in aqueous sodium hydroxide (prepared by dissolving 36.6 g of BPA in an aqueous solution prepared by dissolving 18.4 g of NaOH and 73 mg of sodium dithionite in 269 mL of water) was added to the resultant, and the mixture was subjected to a polymerization reaction for 50 minutes.

200 mL of methylene chloride was added to the resultant for dilution, and the mixture was stirred for 10 minutes. After that, the mixture was separated into an organic phase containing a polycarbonate resin, and an aqueous phase containing excess amounts of bisphenol A and NaOH, and the organic phase was isolated. The resultant solution of the polycarbonate resin in methylene chloride was sequentially washed with 15 vol% each of 0.03 mol/L aqueous NaOH and 0.2 mol/L hydrochloric acid with respect to the solution, and was then repeatedly washed with pure water until an electric conductivity in the aqueous phase after the washing became 0.05 μS/m or less. The solution of the polycarbonate resin in methylene chloride obtained by the washing was concentrated and pulverized, and the resultant flake was dried under reduced pressure at 100°C to provide the polycarbonate resin. The resultant polycarbonate resin was evaluated for the end composition of the polycarbonate resin derived from m-PDP used as an end terminator, the viscosity-average molecular weight (Mv), the flow value (Q value), and the thin-wall moldability. The results are shown in Table 1.

Reference Example 11

The same operations as those of Reference Example 10 were performed except that in the section (2) of Reference Example 10, the amount of m-PDP was changed to 10.1 g. The results of the measurements are shown in Table 1.

Reference Example 12

The same operations as those of Reference Example 10 were performed except that in the section (2) of Reference Example 10, the amounts of methylene chloride, m-PDP, triethylamine, and 6.4 mass% aqueous sodium hydroxide were changed to 55 mL, 7.9 g, 134.5 μL, and 40.3 g, respectively, and the solution of BPA in aqueous sodium hydroxide was changed to a solution prepared by dissolving 33 g of BPA in an aqueous solution prepared by dissolving 16.7 g of NaOH and 66 mg of sodium dithionite in 245 mL of water. The results of the measurements are shown in Table 1.

Comparative Example 1

The same operations as those of Reference Example 8 were performed except that in Reference Example 8, 2.61 g of PTBP was used instead of m-PDP, the amount of triethylamine was changed to 35 μL, and the solution of BPA in aqueous sodium hydroxide was changed to a solution prepared by dissolving 25.7 g of BPA in an aqueous solution prepared by dissolving 15 g of NaOH and 51 mg of sodium dithionite in 219 mL of water. The results of the measurements are shown in Table 1.

Comparative Example 2

The same operations as those of Comparative Example 1 were performed except that in the section (2) of Comparative Example 1, the amount of PTBP was changed to 3.3 g. The results of the measurements are shown in Table 1.

Table 1

| | | Reference Example | | | | | | | | | | | | Comparator Example | |
|---|---|---|---|---|---|---|---|---|---|---|---|---|---|---|---|
| | | 1 | 2 | 3 | 4 | 5 | | 6 | 7 | 8 | 9 | 10 | 11 | 1 | 2 |
| m-PDP | mol% | 6.87 | 5.63 | 4.82 | 0.95 | 1.93 | 2.86 | 4.03 | 3.08 | 3.03 | 5.58 | 5.99 | 4.53 | 0 | 0 |
| PTBP | mol% | 1.44 | 2.88 | 3.23 | 5.84 | 4.54 | 3.17 | 2.43 | 2.91 | 3.04 | 0 | 0 | 0 | 5.88 | 6.71 |
| m-PDP/PTBP | mol/mol | 4.77 | 1.95 | 1.49 | 0.16 | 0.43 | 0.9 | 1.66 | 1.06 | 1 | 1 | 1 | 1 | - | - |
| Mv | | 8,700 | 10,400 | 11,800 | 12,700 | 12,600 | 12,900 | 13,100 | 14,500 | 15,600 | 14,500 | 14,000 | 17,000 | 14,100 | 12,500 |
| Q value (280°C) | $\times 10^{-2}$mL/s | 165 | 150 | 142 | 72 | 75.1 | 64.3 | 90 | 50 | 62.5 | 72 | 85 | 37 | 32 | 40 |
| Thin-wall moldability | | AA | AA | AA | A | A | A | AA | A | A | A | AA | B | X | B |

Example 1

(1) Purification of 3-Pentadecylphenol (m-PDP)

A rectifying column was obtained by filling a column having an inner diameter of 30 mm and a volume of 500 mL with 6-mm Mc. MAHON Packing, and was mounted to a 2 - L flask mounted with an internal temperature-measuring device. The top of the filled column was mounted with an instrument for adjusting a reflux ratio (reflux amount/distillation amount) and a device for measuring the temperature of the column top, and a pressure reduction degree-adjusting device. 1,006.96 g of hydrogenated cardanol manufactured by Tokyo Chemical Industry Co., Ltd. [3-pentadecylphenol: 92.10 mass%, resorcinol derivative: 2.15 mass%, phenol derivative: 5.11 mass%] was supplied to the flask, and the flask was purged with nitrogen, followed by the initiation of heating and a pressure reduction. A pressure reduction degree and the ratio "reflux amount/distillation amount" were set to 2 mmHg and 1, respectively, and a fraction having a column top temperature of from 205°C to 210°C was fractionated. At this time, a temperature in the flask was from 230°C to 245°C. A fractionation amount was 825.71 g (82% of the loading amount) and the purity of 3-pentadecylphenol was 93.61%.

Next, the resultant crude 3-pentadecylphenol (purity: 93.61%) was melted in a hot water bath at 60°C, and 70 g of the molten product was weighed in a standard bottle. After that, 420 g of n-hexane was added to the bottle to dissolve the molten product. The solution was left at rest at room temperature for 12 hours and the precipitated solid was filtered under reduced pressure. After that, the filtrate was dried at room temperature for 8 hours under reduced pressure to provide 48 g of the corresponding 3-pentadecylphenol having a purity of 97.75 mass%. At this time, the 3-pentadecylphenol contained 0.03 mass% of the resorcinol derivative and 2.04 mass% of the phenol derivative as impurities.

70 g of the 3-pentadecylphenol having a purity of 97.75 mass% obtained by the foregoing method was melted in a hot water bath at 60°C, and 70 g of the molten product was weighed in a standard bottle. After that, 420 g of n-hexane was added to the bottle to dissolve the molten product. The solution was left at rest at room temperature for 12 hours and the precipitated solid was filtered under reduced pressure. After that, the filtrate was dried at room temperature for 8 hours under reduced pressure to provide 54 g of 3-pentadecylphenol having a purity of 99.33 mass%. At this time, the 3-pentadecylphenol contained 0.07 mass% of the resorcinol derivative and 0.28 mass% of the phenol derivative as impurities.

(2) Production of Polycarbonate Resin for Liquid Crystal Member

18 L of the oligomer solution obtained in the section (1) of Reference Example 1 and 10.1 L of methylene chloride were loaded into a vessel-type reactor mounted with a baffle board and a paddle-type stirring blade, and having an internal volume of 50 L, and 381 g of 3-pentadecylphenol (m-PDP) having a purity of 97.75 mass% obtained in the section "(1) Purification of 3-Pentadecylphenol (m-PDP)" described above was dissolved in the mixture, followed by the addition of 5 mL of triethylamine to the solution. 1.6 kg of 6.4 mass% aqueous sodium hydroxide was added to the mixture under stirring, and the whole was subjected to a reaction for 10 minutes. Next, a solution of BPA in aqueous sodium hydroxide (prepared by dissolving 1. 3 kg of BPA in an aqueous solution prepared by dissolving 665 g of NaOH and 2.6 g of sodium dithionite in 9.7 L of water) was added to the resultant, and the mixture was subjected to a polymerization reaction for 50 minutes.

Methylene chloride was added to the resultant for dilution, and the mixture was stirred for 10 minutes. After that, the mixture was separated into an organic phase containing a polycarbonate resin generated through the polymerization reaction, and an aqueous phase containing excess amounts of bisphenol A and NaOH, and the organic phase was isolated. The resultant solution of the polycarbonate resin in methylene chloride (organic phase) was sequentially washed with 15 vol% each of 0.03 mol/L aqueous NaOH and 0.2 mol/L hydrochloric acid with respect to the solution, and was then repeatedly washed with pure water until an electric conductivity in the aqueous phase after the washing became 0.05 μS/m or less. The solution of the polycarbonate resin in methylene chloride obtained by the washing was concentrated and pulverized, and the resultant flake was dried under reduced pressure at 100°C to provide the polycarbonate resin. The $^1$H-NMR measurement of the resin showed that the composition amount of an end group derived from m-PDP was 4.53 mol%, the composition amount of an end group derived from PTBP was 2.92 mol%, and the composition amount of an end group derived from OH was 0.03 mol%, and the amount of an unreacted PDP in the resin was 6 ppm by mass. The resultant polycarbonate resin had a viscosity-average molecular weight (Mv) of 11,900 and a flow value (Q value) at 280°C of 123 ($\times 10^{-2}$ mL/sec), and its thin-wall moldability was evaluated as AA. In addition, the YI of the resultant polycarbonate resin for a liquid crystal member was measured to be 1.1.

In addition, the total light transmittance of the resultant polycarbonate resin for a liquid crystal member at a wavelength of 400 nm was measured to be 88.1%.

Example 2

A polycarbonate resin for a liquid crystal member was produced in the same manner as in the section " (2) Production of Polycarbonate Resin for Liquid Crystal Member" of Example 1 except that in the section (2) of Example 1, the 3-pentadecylphenol (m-PDP) having a purity of 99.33 mass% obtained in the section (1) of Example 1 was used instead of the 3-pentadecylphenol having a purity of 97.75 mass%. The $^1$H-NMR measurement of the resultant polycarbonate resin for a liquid crystal member showed that the composition amount of an end group derived from m-PDP was 4.54 mol%, the composition amount of an end group derived from PTBP was 2.69 mol%, and the composition amount of an end group derived from OH was 0. 04 ml%, and the amount of an unreacted PDP in the resin was 6 ppm by mass. The polycarbonate resin for a liquid crystal member had a viscosity-average molecular weight (Mv) of 11, 500 and a flow value (Q value) of 127 ($\times 10^{-2}$ mL/sec), and its thin-wall moldability was evaluated as AA. In addition, the YI of the resultant polycarbonate resin for a liquid crystal member was measured to be 1.0.

In addition, the total light transmittance of the resultant polycarbonate resin for a liquid crystal member at a wavelength of 400 nm was measured to be 88.6%.

Comparative Example 3

A polycarbonate resin for a liquid crystal member was produced in the same manner as in the section " (2) Production of Polycarbonate Resin for Liquid Crystal Member" of Example 1 except that in the section (2) of Example 1, the 3-pentadecylphenol having a purity of 92.10 mass% [manufactured by Tokyo Chemical Industry Co. , Ltd.] was used instead of the 3-pentadecylphenol having a purity of 97.75 mass%. The $^1$H-NMR measurement of the obtained resultant resin for a liquid crystal member showed that the composition amount of an end group derived from m-PDP was 4.55 mol%, the composition amount of an end group derived from PTBP was 2.90 mol%, and the composition amount of an end group derived from OH was 0.06 mol%, and the amount of an unreacted PDP in the resin was 7 ppm by mass. The polycarbonate resin for a liquid crystal member had a viscosity-average molecular weight (Mv) of 12, 000 and a flow value (Q value) of 123 ($\times 10^{-2}$ mL/sec), and its thin-wall moldability was evaluated as AA. In addition, the YI of the resultant polycarbonate resin for a liquid crystal member was measured to be 4.6.

In addition, the total light transmittance of the resultant polycarbonate resin for a liquid crystal member at a wavelength of 400 nm was measured to be 81.7%.

Each of the polycarbonate resins for liquid crystal members obtained in Reference Examples 1 to 12, and Examples 1 and 2 has a high flow value (Q value) and is particularly excellent in thin-wall moldability, and hence can be suitably used as a polycarbonate resin for a liquid crystal member. In addition, as can be seen from Examples 1 and 2, when high-purity m-PDP is used, a polycarbonate resin for a liquid crystal member having a low YI and excellent in transparency can be obtained, and can be suitably used in, for example, a light-guiding plate or a light-diffusing plate.

INDUSTRIAL APPLICABILITY

The polycarbonate resin for a liquid crystal member of the present invention is excellent in flowability and color tone, and is hence excellent in moldability and suitable for the production of, in particular, a molded body having a small thickness, a light-guiding plate or light-diffusing plate for a liquid crystal display apparatus, or any other OA equipment material member.

## Claims

1. A polycarbonate resin for a liquid crystal member, which is produced by using an end terminator comprising 3-pentadecylphenol obtained from a natural product, the polycarbonate resin having a YI value in the following measurement method of 1.1 or less:

<Method of measuring YI Value>

500 ppm by mass of bis(2,6-di-t-butyl-4-methylphenyl)pentaerythritol phosphite is added to the polycarbonate resin, the mixture is melted, kneaded, and extruded with a vented single screw extruder of 40 mmφ at a resin temperature of 280°C and a screw rotation speed of 100 rpm to provide a pellet, the resultant pellet is molded into a molded article having a thickness of 3 mm, and the molded article is subjected to measurement with a spectrophotometer by a transmission method at a measurement area of 30 φ under a C2 light source.

**2.** A polycarbonate resin for a liquid crystal member, which is produced by using an end terminator comprising 3-pentadecylphenol obtained from a natural product, the polycarbonate resin having a light transmittance at a wavelength of 400 nm in the following measurement method of 85% or more:

<Method of measuring Light Transmittance at Wavelength of 400 nm>

500 ppm by mass of bis(2,6-di-t-butyl-4-methylphenyl)pentaerythritol phosphite is added to the polycarbonate resin, the mixture is melted, kneaded, and extruded with a vented single screw extruder of 40 mmφ at a resin temperature of 280°C and a screw rotation speed of 100 rpm to provide a pellet, the resultant pellet is molded into a molded article having a thickness of 3 mm, and the molded article is subjected to measurement of a total light transmittance with a spectral photometer.

**3.** The polycarbonate resin for a liquid crystal member according to claim 1 or 2, wherein a composition amount of an end group derived from OH with respect to all end groups in the polycarbonate resin is 5.0 mol% or less.

**4.** The polycarbonate resin for a liquid crystal member according to any one of claims 1 to 3, wherein the 3-pentadecylphenol has a purity of 97.5 mass% or more.

**5.** The polycarbonate resin for a liquid crystal member according to any one of claims 1 to 3, wherein the 3-pentadecylphenol has a purity of 97.75 mass% or more.

**6.** The polycarbonate resin for a liquid crystal member according to any one of claims 1 to 3, wherein the 3-pentadecylphenol has a purity of 99.33 mass% or more.

**7.** The polycarbonate resin for a liquid crystal member according to any one of claims 1 to 6, wherein the polycarbonate resin is produced by using an end terminator comprising 3-pentadecylphenol obtained by performing distillation and crystallization.

**8.** The polycarbonate resin for a liquid crystal member according to claim 7, wherein the crystallization is performed after the distillation.

**9.** The polycarbonate resin for a liquid crystal member according to claim 7 or 8, wherein the crystallization comprises using a hydrocarbon-based solvent.

**10.** The polycarbonate resin for a liquid crystal member according to claim 9, wherein the hydrocarbon-based solvent to be used in the crystallization comprises one or more of hexane and heptane.

**11.** The polycarbonate resin for a liquid crystal member according to any one of claims 7 to 10, wherein the crystallization comprises using a solvent in an amount of 2 parts by mass or more and 20 parts by mass or less with respect to 1 part by mass of the 3-pentadecylphenol.

**12.** The polycarbonate resin for a liquid crystal member according to any one of claims 7 to 10, wherein the crystallization comprises using a solvent in an amount of 4 parts by mass or more and 10 parts by mass or less with respect to 1 part by mass of the 3-pentadecylphenol.

**13.** The polycarbonate resin for a liquid crystal member according to any one of claims 1 to 12, wherein the end terminator further comprises p-t-butylphenol or p-cumylphenol.

**14.** Apolycarbonate resin composition for a liquid crystal member, comprising:

the polycarbonate resin for a liquid crystal member of any one of claims 1 to 13; and
an aromatic polycarbonate resin except the polycarbonate resin.

**15.** A liquid crystal member, which is produced by molding the polycarbonate resin for a liquid crystal member of any one of claims 1 to 13, or the polycarbonate resin composition for a liquid crystal member of claim 14.

**16.** A polycarbonate resin for a light-guiding plate, wherein the polycarbonate resin for a liquid crystal member of any

one of claims 1 to 13 is used for a light-guiding plate.

**17.** A polycarbonate resin composition for a light-guiding plate, comprising:

the polycarbonate resin for a light-guiding plate of claim 16; and
an aromatic polycarbonate resin except the polycarbonate resin.

**18.** A light-guiding plate, which is produced by molding the polycarbonate resin for a light-guiding plate of claim 16 or the polycarbonate resin composition for a light-guiding plate of claim 17.

**19.** A method of producing a polycarbonate resin for a liquid crystal member, comprising using 3 -pentadecylphenol obtained from a natural product and having a purity of 97.5 mass% or more as an end terminator.

**20.** The method of producing a polycarbonate resin for a liquid crystal member according to claim 19, wherein the 3 -pentadecylphenol has a purity of 97.75 mass% or more.

**21.** The method of producing a polycarbonate resin for a liquid crystal member according to claim 19 or 20, wherein the 3-pentadecylphenol has a purity of 99.33 mass% or more.

**INTERNATIONAL SEARCH REPORT**

International application No.
PCT/JP2014/060926

A. CLASSIFICATION OF SUBJECT MATTER
*C08G64/14*(2006.01)i, *C07C39/06*(2006.01)i, *F21S2/00*(2006.01)i, *G02F1/1333* (2006.01)i

According to International Patent Classification (IPC) or to both national classification and IPC

B. FIELDS SEARCHED

Minimum documentation searched (classification system followed by classification symbols)
C08G64/14, C07C39/06, F21S2/00, G02F1/1333

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2004-331688 A (Teijin Chemicals Ltd.), 25 November 2004 (25.11.2004), claims; paragraphs [0028], [0046], [0072]; examples (Family: none) | 1-18<br>19-21 |
| Y | JP 2004-507585 A (General Electric Co.), 11 March 2004 (11.03.2004), claims; paragraphs [0016], [0017] & US 6255439 B1 & EP 1315692 A1 & WO 2002/018312 A1 & DE 60130345 T & AU 6858001 A & CN 1449367 A | 19-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 May, 2014 (02.05.14) | 13 May, 2014 (13.05.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/060926

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-511811 A (General Electric Co.), 28 April 2005 (28.04.2005), the entire specification & US 2003/0105271 A1 & EP 1436342 A1 & WO 2003/048230 A1 & DE 60213270 T & KR 10-2005-0035139 A & CN 1697849 A & AU 2002351483 A | 1-21 |
| A | WO 2011/096089 A1 (Teijin Ltd.), 11 August 2011 (11.08.2011), the entire description & DE 102009027836 A | 1-21 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 52050078 B
- JP 2003041011 A
- JP 2662310 B